# EUROPEAN PATENT APPLICATION

(11) **EP 4 667 907 A1**
(43) Date of publication of application: **24.12.2025**
(21) Application number: 24188471.7
(22) Date of filing: 12.07.2024
(51) Int. Cl.: G01N 21/25, G01N 21/47, G01N 21/3563, G01N 21/359, G01N 33/36, G01N 21/84

(54) **DEVICE AND METHOD FOR IDENTIFYING STRUCTURE AND COMPOSITION OF A FABRIC OR TEXTILE**

(30) Priority: 21.06.2024 PT 2024119539
(71) Applicant: Casa da Malha - C5M, Lda, 4750-625 Barcelos (PT); International Iberian Nanotechnology Laboratory (INL), 4715-330 Braga (PT)
(72) Inventor: DA SILVA MARTINS, JOÃO MARCO, 4715-330 BRAGA (PT); PINTO DOS SANTOS MOURA, JOÃO MARTINHO, 4715-330 BRAGA (PT); DE ALMEIDA RODRIGUES, JOÃO MANUEL, 4715-330 BRAGA (PT); DE SOUSA BARROS DA MOTA, DUARTE NUNO, 4715-330 BRAGA (PT); DE ABREU PEDRO, ADRIANO JOSÉ, 4715-330 BRAGA (PT); FERNANDES DA COSTA, FILIPA JOÃO, 4750-625 BARCELOS (PT); CARRINHO RIBEIRO, PEDRO JOSÉ, 4715-330 BRAGA (PT)
(74) Representative: Patentree

(57) **Abstract**

Device for identifying structure and composition of a fabric or textile, comprising: a chamber placing over the fabric or textile, with a microscopic camera attached to the chamber, for acquiring an image of the fabric or textile, , a chamber diffused light for illuminating the fabric or textile with diffused light reflected by the chamber, a chamber grazing light, for illuminating the fabric or textile with grazing light, and a rotating mechanism to orient the microscopic camera with the fabric woven alignment; a near infrared spectrometer attached to the surface plate where the fabric is set, to capture an absorption spectrum in multiple wavelengths, ranging from 1550-1950 nm, a white reference aligned over the near infrared spectrometer to calibrate the absorption spectrum and reduce the thickness influence of the fabric; an electronic system for light control, a software for interfacing and control of the acquisition and recognition processes, by carrying out the following steps: activating the chamber diffused light and the chamber grazing light; acquiring, from the microscopic camera, the acquired image; activating the near-infrared light source, acquiring, from the near-infrared spectrometer, the absorption spectrum; and detecting the structural identification and the composition of the textile or fabric, from the acquired image and the near infrared spectrum.

## Description

### TECHNICAL FIELD

The present disclosure relates to a device and method for identifying structure and composition of a fabric or textile, comprising a chamber for placing over the fabric or textile on a surface, a diffused-light emitter for illuminating the chamber with diffuse light; a grazing-light emitter for illuminating the fabric or textile with grazing light; a camera for acquiring images of the fabric or textile, mounted inside the chamber; a near-infrared spectrometer for acquiring a near-infrared spectrum of the fabric ortextile; and a data processor; wherein the device is configured for acquiring images of the fabric or textile at a plurality of rotation angles of the fabric or textile about an axis perpendicular to the surface, for classifying the structural identification and the composition of the textile or fabric, from the acquired image and the acquired spectrum.

### BACKGROUND

The textile industry consistently introduces a plethora of new and innovative fabrics featuring diverse compositions, structures, and string parameters.

Currently, the examination of these fabrics requires the expertise of a skilled individual through a manual inspection process. This inspection is not only time-consuming and often destructive but also demands a wealth of accumulated knowledge and experience, which is presently challenging to find in the labour market.

When examining these tasks individually, we identify existing commercial solutions for composition analysis. Conversely, no commercially available solution is currently found for structure analysis. In this context, we will showcase the prevailing solutions for each case. In terms of (1) Composition Analysis, we will enumerate commercially available solutions. On the other hand, for (2) Structure Analysis, given the absence of a commercially available solution, we will introduce solutions put forth by the research community.

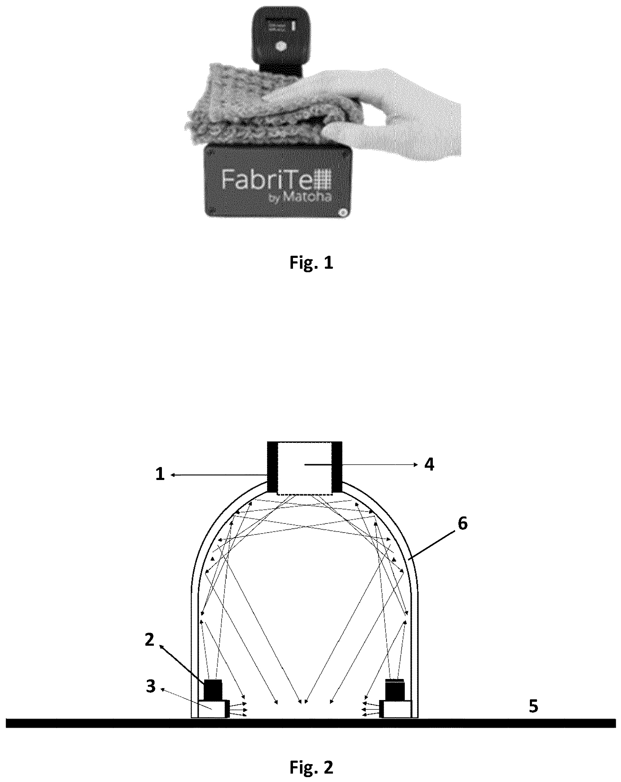

**Figure 1** shows an example of Matoha instrument to measure composition of fabrics. Most of the existing technology for composition analysis follows the usage of a NIR spectrometer as a standalone, portable device. It allows to take a spectrum of a fabric sample by placing the fabric on top of the spectrometer. From the pattern of the absorption spectrum, it is possible to retrieve information about its composition. Several pure materials and some of dual blends are identified by the system.

FiberSort is a company that uses NIR hyperspectral cameras to sort monomaterial fabrics for recycling purposes. It uses the same principle than Matoha as well as the one we are using in the proposed innovation, but instead, uses the surface cameras. This system performs fabric sorting by composition for recycling purposes.

Limitations of the prior art: (1) The identified pure spectrums lack the capability to discriminate between subcategories that have near identical chemical composition. Notably, fabrics primarily composed of cellulose or viscose (regenerated cellulose) cannot be differentiated by this system. Consequently, materials such as cotton and linen (both seed cellulose-based) are indistinguishable, and subcategories within viscose (CV and lyocell) also remain inseparable. Given the substantial cost disparity between cotton and linen, misclassification of these materials could result in significant production costs. (2) Complex mixtures/blends of materials (3 or more) are hard to accurately classify. The principal reason is the lack of physical data available for all possible blend ratios.

US11429822B2 discloses a fabric identifying system including a fabric identifying apparatus for identifying the type of a fabric of clothing and a server. The fabric identifying apparatus includes an image camera for obtaining image information on a fabric structure of clothing, a fabric identifier for performing a function of identifying the type of the fabric based on the fabric structure of the image information. The server includes an artificial intelligence model learner for generating a fabric type identifying engine for learning the fabric structure of the image information of the received clothing through a deep neural network, the server is configured to transmit the learned fabric type identifying engine to the fabric identifying apparatus. According to the present disclosure, it is possible to identify the type of the fabric of the clothing by using the artificial intelligence (Al), the artificial intelligence based screen recognition technology, and the 5G network.

Patent document CN108645814B discloses a collecting method of a hyperspectral image for recognizing a wetted area of a colourful fabric. The collecting method is characterized in that an EVA (ethylene vinyl acetate)foam improved clamping device is used for providing a precise division boundary for the image extracting of a tested area; the hyperspectral image is a gray level image set at a plurality of wave band channels, the gray level images at the near-infrared wave band can be extracted, and the image brightness value is unrelated with R (red), G (green) and B (blue) components, so as to effectively overcome the influence by the fabric color; direct current is used for supplying power for a diffuse reflection type halogen light source, and the intensity of light provided by a light source is stable; a motor is used for pushing a specimen on a specimen carrying box at uniform speed through an electrically-controlled conveying belt so as to enable a CCD (charge coupled device) camera to scan by lines, and the light intensity in the scanning process of the fabric each time is the same, and is equivalent to the uniform light intensity of the fabric surface; after the image is collected by a hyperspectral image collecting system, white board correction and dark current correction methods are adopted, and the influence by the nonuniform light radiation at the image surface of the original fabrics further eliminated; the wetted area and the unwetted area have better contrast degree in the gray level image at the infrared waveband, and the influence by the texture of the fabric surface can be effectively overcome by proper resolution ratio.

There is currently no commercially available technology designed for the structure analysis of woven fabrics. Although various scientific literature contains works proposing strategies for such analyses and recognition tasks, none have transitioned into commercial solutions. Below, relevant works are enumerated that approximate the strategy utilized in the proposed invention and provide context regarding the vision-based strategies employed to address this problem.

The disadvantages of the mentioned solution for composition analysis are related with: (1) only surface analysis of fabric (making the general analysis more difficult), (2) blends of up to 2 elements, and no more than that, (3) subcategories are not distinguishable (linen vs cotton vs hemp, or viscose vs lyocell).

Regarding structure analysis, (1) lightning conditions were not perfectly defined for all colour types and scales, and (2) expensive equipment was necessary for high-quality pictures.

These facts are disclosed in order to illustrate the technical problem addressed by the present disclosure.

### GENERAL DESCRIPTION

The present disclosure aims to classify the structural identification and composition of textile or fabric based on acquired images and spectra.

The proposed disclosure aligns itself within the realm of digital transformation in the textile industry, addressing the imperative need for automating the fabric inspection process. Specifically, it aims to automatically extract the most pertinent characteristics of an unknown fabric sample, mitigating the current challenges associated with manual inspection.

This endeavour encompasses two primary technical tasks: (1) fabric structure analysis and (2) composition analysis.

To achieve these objectives, the proposed disclosure employs two key instruments: (1) a camera, in particular a microscopic camera, with custom light system for structure identification and (2) a near-infrared spectrometer (NIR) for composition analysis.

In general, vision-based methods are used in this context to retrieve fabric structural parameters, such as weave and weft patterns, density, yarn counts, and colour patterns [5]. So far, the systems developed focus on a specific part of the fabric structure or parameters. In the context in which the proposed invention is being developed, the users will tryto estimate parameters from unknown samples and should be able to increasingly add new structures and novel material, following the fast-paced change of trends in the textile industry.

Most vision systems have the same type of hardware: (1) camera, (2) lens, and (3) light [5]. Different types of cameras can be used depending on the application: area-scan or line-scan based cameras, being the first type, more commonly applied. The settings of the vision system can change but most follow a similar approach with fixed distance and pre-set magnification.

The techniques typically utilize a microscope positioned at a specific distance to capture standard images of fabrics. However, the lighting conditions, processing pipelines, and algorithms employed in these works vary significantly. The primary sequence of actions for structure classification involves: (1) employing an image system to capture surface images of fabrics, (2) pre-processing the images, and (3) implementing a classification model.

In each of these stages, notable variations are observed. For the proposed disclosure, optimal conditions were achieved in the first and third stages, eliminating the need for pre-processing. This optimization yielded several benefits that highlight the proposed disclosure from the other identified systems in the literature review.

The automated systems found in the literature include (1) single-sided imaging systems [1, 6, 7, 8] (2) dual-side imaging systems [9, 10], (3) backlighting systems [11, 12] and (4) multi-light systems [13]. The limitations of each of them is listed below:
(1) - Less informative, as the structure can depend on front and back pictures;
(2) - The operation is considered more complex; the processing time is longer and more physical space is required;
(3) - Transparency makes the image lose relevant information of the structure
(4) - More expensive, longer processing time and more physical-space is required.

Disclosed herein is a purpose embodiment of a device for identifying structure and composition of a fabric or textile, comprising: a chamber placed over the fabric or textile; a camera, in particular a microscopic camera, attached to the chamber, for acquiring an image of the fabric or textile, a near-infrared spectrometer attached to the chamber, for acquiring a near infrared spectrum of the fabric or textile, which shows an intensity of light at different wavelengths in the near-infrared range; a chamber diffused light for illuminating the fabric or textile with diffused light reflected by the chamber; a chamber grazing light, for illuminating the fabric or textile with grazing light; an electronic data processor configured for identifying the structure and the composition of the fabric or textile, by carrying out the steps of: activating the chamber diffused light and the chamber grazing light; acquiring, from the microscopic camera, the acquired image; acquiring, from the near-infrared spectrometer, the near infrared spectrum; and detecting the structural identification and the composition of the textile or fabric, from the acquired image and the near infrared spectrum.

It is disclosed a device for identifying structure and composition of a fabric or textile, comprising: a chamber for placing over the fabric or textile on a surface, a diffused-light emitter for illuminating the chamber with diffuse light; a grazing-light emitter for illuminating the fabric or textile with grazing light; a camera for acquiring images of the fabric or textile, mounted inside the chamber; a near-infrared spectrometer for acquiring a near-infrared spectrum of the fabric or textile; and a data processor; wherein the device is configured for acquiring images of the fabric or textile; wherein the data processor is arranged for carrying out the following steps: activating the diffused-light emitter and the grazing-light emitter; acquiring, from the camera, an image of the fabric or textile; wherein the data processor is further arranged for carrying out the following steps: acquiring, from the near-infrared spectrometer, a near-infrared spectrum of the fabric or textile; using a pre-trained classifier for classifying the structural identification and the composition of the textile or fabric, from the acquired image and the acquired spectrum.

In an embodiment, the device is further configured for acquiring images of the fabric or textile at a plurality of rotation angles of the fabric or textile about an axis perpendicular to the surface.

In an embodiment, the chamber can comprise a rotation union for rotating, relative to the fabric or textile, a part of the chamber where camera is mounted.

In an embodiment, the chamber is rotatable by a user of the device, in particular the chamber comprising a manual rotating mechanism for rotating the camera or the part of the chamber where the camera is mounted, further in particular the chamber comprising a handle for rotating the camera or the part of the chamber where the camera is mounted.

In an embodiment, the data processor is further arranged for detecting misalignment in the acquired image of the fabric or textile, between a structural direction of the fabric or textile and a reference direction, and if misalignment is detected, alerting (i.e., guiding) a user of the device for re-acquiring, from the camera, an image of the fabric or textile rotated in respect of the previously acquired image about an axis perpendicular to the surface for aligning the structural direction of the fabric or textile with the reference direction.

In an embodiment, the data processor is further arranged for: detecting misalignment in the acquired image of the fabric or textile, between a structural direction of the fabric or textile and a reference direction; if misalignment is detected, actuating the motor, in particular a rotating mechanism, for rotating the camera or the part of the chamber where camera is mounted for aligning the structural direction of the fabric or textile with the reference direction; and re-acquiring, from the camera, an image of the fabric or textile rotated in respect of the previously acquired image about an axis perpendicular to the surface.

In an embodiment, the data processor is further arranged for: detecting misalignment in the acquired image of the fabric or textile, between a structural direction of the fabric or textile and a reference direction; if misalignment is detected, using digital image processing for rotating the acquired image for aligning the structural direction of the fabric or textile with the reference direction.

In an embodiment, the data processor is arranged for adjusting light intensity of the diffused-light emitter and the grazing-light emitter such that the acquired image of the fabric or textile falls within a target brightness range. Adjusting the intensity of the dome light and the grazing light until the image of the fabric or textile is inside a target brightness range, such that all acquired images have a similar overall brightness, independently of their colour.

In an embodiment, the data processor is further arranged for carrying out the preparatory step of acquiring a white reference signal for near-infrared calibration.

In an embodiment, the chamber is at least partially a hemispherical diffusing chamber, in particular a portion of the chamber is hemispherical.

In an embodiment, the camera can be rotatably mounted inside the chamber and can be rotatable.

In an embodiment, the chamber can comprise a rotation union for rotating, relative to the fabric or textile, a part of the chamber where the camera is mounted.

In an embodiment, the camera can be rotatable by a user of the device, in particular the chamber comprising a manual rotating mechanism, further in particular the chamber comprising a handle for rotating the camera or the part of the chamber.

In an embodiment, the device can comprise a rotating mechanism for microscopic image device alignment or the part of the chamber where camera is mounted.

In an embodiment, the processor can be configured for rotating a captured image.

In an embodiment, the diffuse-light emitter can comprise an illuminator ring or dome for illuminating the chamber with light to be reflected by the inner surface of the chamber.

In an embodiment, the grazing-light emitter can be mounted inside the chamber at the bottom of the chamber.

In an embodiment, the grazing-light emitter can comprise an illuminator ring for illuminating fabric or textile with grazing light.

In an embodiment, the camera can be a microscopic camera.

In an embodiment, the device may further include a platform surface on which the fabric or textile is placed.

In an embodiment, the platform surface comprises the near-infrared spectrometer arranged for acquiring a near-infrared spectrum of the fabric or textile placed over the platform surface

In an embodiment, the device can comprise a pressing surface for pressing the fabric or textile over the platform surface and wherein the chamber is mounted on the pressing surface.

In an embodiment, the pressing surface comprises a reflective white reference for calibrating the near-infrared spectrometer.

In an embodiment, the reflective white reference and the near-infrared spectrometer are mounted opposite and aligned to each other.

In an embodiment, the chamber is a dome-shaped cover or a hemisphere - shaped cover.

In an embodiment, the data processor is further arranged for adjusting light source intensity of at least one of the diffused-light emitter and the grazing-light emitter, in particular according to the fabric or textile colour.

In an embodiment, the data processor is arranged to, simultaneously or subsequently, acquire the camera image and the near-infrared spectrum.

In an embodiment, the camera has a fixed focal length and magnification.

The machine learning method was carried out with the support of data aggregation, labelling, and material delivering. CM provided physical samples of knitwear of various compositions and structures and technical data on them, including description, composition, yarns, incorporations, LFA, grammage, width, etc.

In an embodiment, human/user classification is linked to the computer-based classification by way of a cloud system and storage. Classification can be done locally inside device. Cloud system can be used for synchronization

In an embodiment, the machine learning method involves incremental learning enhanced by human supervision.

It is disclosed a computer-based method for identifying structure and composition of a fabric or textile using a device according to any of the previous embodiments, the method comprising the following steps: activating and automatically adjusting the intensity of the diffused-light emitter and the intensity of the grazing-light emitter; acquiring, from the camera, an image of the fabric or textile; if misalignment is detected in the acquired image, re-acquiring, from the camera, an image of the fabric or textile rotated in respect of the previously acquired image about an axis perpendicular to the surface; wherein the method is further arranged for carrying out the following steps: acquiring, from the near-infrared spectrometer, a near-infrared spectrum of the fabric or textile; using a pretrained classifier for classifying the structural identification and the composition of the textile or fabric, from the acquired image and the acquired spectrum.

Non-transitory storage media including program instructions for implementing a computer-based method for identifying structure and composition in a fabric or textile, the program instructions including instructions executable by a data processor to carry out the method described in the previous embodiment.

### References

[1] Zhigang Xia, Mian Zhou, Hongshan Wang, Kezuo Wang & Youshun Wan. (2022) Evaluating the surface hairiness of woven fabric belts with a yarn hairiness tester. The Journal of The Textile Institute 113:1, pages 116-124.
[2] Shen, J., Zou, X., Xu, F., Xian, Z. (2010). Intelligent Recognition of Fabric Weave Patterns Using Texture Orientation Features. In: Zhu, R., Zhang, Y., Liu, B., Liu, C. (eds) Information Computing and Applications. ICICA 2010. Communications in Computer and Information Science, vol 106. Springer, Berlin, Heidelberg. https://doi.org/10.1007/978-3-642-16339-5_2.
[3] Ali Moussa, Daniel Dupont, Daniel Steen & Xianyi Zeng (2010) Structure analysis and surface simulation of woven fabrics using fast Fourier transform techniques, The Journal of The Textile Institute, 101:6, 556-570, DOI: 10.1080/00405000802596958.
[4] Meng, S., Pan, R., Gao, W. et al. A multi-task and multi-scale convolutional neural network for automatic recognition of woven fabric pattern. J Intell Manuf 32, 1147-1161 (2021). https://doi.org/10.1007/s10845-020-01607-9.
[5] - Meng, S., Pan, R., Gao, W. et al. Automatic recognition of woven fabric structural parameters: a review. Artif Intell Rev 55, 6345-6387 (2022). https://doi.org/10.1007/s10462-022-10156-x.
[6] - Chen, Siqiang et al. 'Image-based Textile Decoding'. 1 Jan. 2021: 177 - 190.
[7] - Iqbal Hussain, M.A.; Khan, B.; Wang, Z.; Ding, S. Woven Fabric Pattern Recognition and Classification Based on Deep Convolutional Neural Networks. Electronics 2020, 9, 1048. https://doi.org/10.3390/electronics9061048.
[8] - Zhitao Xiao, Xiao ting Liu, Jun Wu, Lei Geng, Ying Sun, Fang Zhang & Jun Tong (2018) Knitted fabric structure recognition based on deep learning, The Journal of The Textile Institute, 109:9, 1217-1223, DOl: 10.1080/00405000.2017.1422309.
[9] - Binjie Xin, Jinlian Hu, Baciu G, Xiaobo Yu. Investigation on the Classification of Weave Pattern Based on an Active Grid Model. Textile Research Journal. 2009;79(12):1123-1134. doi:10.1177/0040517508101459. Url: https://journals.sagepub.com/doi/10.1177/0040517508101459.
[10] - Zhang R, Xin B (2016a) An investigation of density measurement method for yarn-dyed woven fabrics based on dual-side fusion technique. Meas Sci Technology 27:085403. Url: https://iopscience.iop.org/article/10.1088/0957-0233/27/8/085403.
[11] - Foruzan Fasahat & Pedram Payvandy (2017) A novel hybrid genetic and imperialist competitive algorithm for structure extraction of woven fabric images, The Journal of The Textile Institute, 108:6, 893-905, DOI: 10.1080/00405000.2016.1197494.
[12] - Lim, J., Kim, S. Analysis of woven fabric structure using image analysis and artificial intelligence. Fibers Polym 12, 1062-1068 (2011). https://doi.org/10.1007/s12221-011-1062-8.
[13] - Jiaping Li, Wendi Wang, Na Deng, Binjie Xin, A novel digital method for weave pattern recognition based on photometric differential analysis, Measurement, Volume 152, 2020, 107336, ISSN 0263-2241, https://doi.org/10.1016/j.measurement.2019.107336.

### BRIEF DESCRIPTION OF THE DRAWINGS

The following figures provide preferred embodiments for illustrating the disclosure and should not be seen as limiting the scope of invention.
**Figure 1****:** Schematic representation of an example of Matoha instrument to measure composition of fabrics.
**Figure 2****:** Schematic representation of an embodiment of the detailed technical light system.
**Figure 3****:** Schematic representation of an embodiment of the software operations.
**Figure 4****:** Schematic representation of a Siamese Convolutional Neural Network and an Ensemble Prediction for structure analyses.
**Figure 5****:** Schematic representation of a Pre-Processing and Modelling, containing an Authenticator and a Regressor, for composition analyses.
**Figure 6****:** Schematic representation of an embodiment of a device for identifying structure and composition of a fabric or textile.

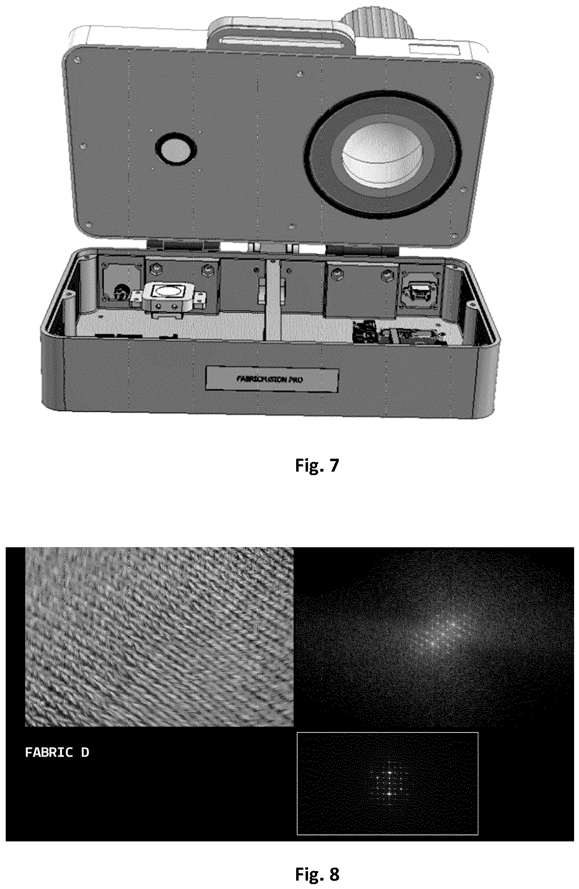

**Figure 7****:** Schematic representation of another perspective view of the embodiment.
**Figure 8****:** Schematic representation of an acquired image and its Inversed Fourier Transform, representing spatial domain.

### DETAILED DESCRIPTION

The proposed disclosure represents a system that conducts both structure and composition analyses.

**Figure 2** shows a detailed technical drawing of a Light System, where it can be seen **1** represents a fine rotation adjustment system; **2** represents a circular dome light; **3** represents a circular grazing light; **4** represents a microscope camera and **5** represents a textile fabric.

In an embodiment, the device for identifying structure and composition of a fabric or textile **5,** can comprise: a chamber **6** for placing over the fabric or textile on a surface, a diffused-light emitter **2** for illuminating the chamber with diffuse light; a grazing-light emitter **3** for illuminating the fabric or textile with grazing light; a camera **4** for acquiring images of the fabric or textile, mounted inside the chamber; a near-infrared spectrometer for acquiring a near-infrared spectrum of the fabric or textile; and a data processor; wherein the device is configured for acquiring images of the fabric or textile at a plurality of rotation angles of the fabric or textile about an axis perpendicular to the surface; wherein the data processor is arranged for carrying out the following steps:
activating the diffused-light emitter and the grazing-light emitter;
acquiring, from the camera, an image of the fabric or textile;
adjusting the intensity of the circular dome light and adjusting of the intensity of the circular grazing light until the diffusing hemispherical chamber light container has the optimal light condition for a final acquiring;
if misalignment is detected in the acquired image, re-acquiring, from the camera,
an image of the fabric or textile rotated in respect of the previously acquired
image about an axis perpendicular to the surface;
wherein the data processor is further arranged for carrying out the following steps:
acquiring, from the near-infrared spectrometer, a near-infrared spectrum of the fabric or textile;
using a pretrained classifier for classifying the structural identification and the composition of the textile or fabric, from the acquired image and the acquired spectrum.

Custom Lightning Conditions. In an embodiment, dome + grazing light can be seen as one of the main distinguished aspects of the proposed disclosure regarding the usage of a custom lightning system that allows perfect conditions for the capture of detailed surface textile pictures. This was possible by using a Dome + Grazing light source. The result is a high-quality picture, unaffected by external light.

In an embodiment, automatic light intensity adjustment allows the optimal brightness of the picture to be held steady within image acquisitions. In order to maintain the optimal brightness conditions as standard as possible, an adaptive light algorithm was developed to control the light source and adjust the light intensity for each textile sample. This technique allows to keep pictures with the optimal brightness independently of their colour.

In an embodiment, compactness of Image Capturing System addresses some of the most commonly reported issues, including light interference and stabilization. The present disclosure, with a custom and compact light system, allows adjustable direction to perform standard capture conditions. All of this enables capturing the perfect picture without the need for expensive cameras, multi light systems or backlighting.

In an embodiment, the two-in-One Structure + Composition Analysis of Textile innovatively captures high-quality pictures of textiles for structure analysis and NIR spectrums for composition analysis. These analyses can be conducted simultaneously or separately.

In an embodiment, the structure and composition of both the front and back fabric are captured through images and spectra. For fabric structure analysis, the device employs an innovative algorithm that utilizes both front and back images to provide accurate estimations of structure classes, while also predicting front and back surface compositions. This feature offers a distinct advantage in structure and composition analysis.

In terms of structure estimation, for the diversity of structures that exist, front and back images are necessary to perform accurate and reliable estimations, as weave patterns differ in front and back. In the case of composition, the previous technologies inspect the fabric as a whole, only inspecting the surfaced faced to the NIR sensor. In this case, it allows the user to inspect each surface of the fabric. Considering the NIR technology only measures the absorption in the surface of the fabric (up to 2 mm inside it) and that the fabric might not be homogeneous in terms of composition, the proposed disclosure gives an estimation of the composition for both faces independently, allowing to give more reliable predictions about the different materials used to build the fabric.

In an embodiment, current composition systems assert that subcategories of cellulose type materials, such as cotton and linen, are indistinguishable due to the high similarity in their chemical composition. This similarity poses a challenge for differentiation using a NIR system. We were able to record signals under conditions that allow for such separability, encompassing both cellulose types (Cotton and Linen) as well as two viscose types (CLY Viscose and CV Viscose).

In one embodiment, the device can comprise an integrated software. A user interface and software are integrated in the device and can be used to select the capture settings or the type of analysis. Results are provided to the user in an intuitive way.

The present disclosure provides a system capable of identifying the structure and composition of fabrics, automatically.

The device has two main sensors: (1) a microscope with a custom light system and (2) a NIR spectrometer.

Regarding the camera, it was used to estimate the structure of the fabric. It requires the collection of front and back fabric pictures. The distance of the camera and focus of the microscope are preset and optimized for the pictures used for the development of the machine learning model. These pictures are high-quality images of the fabric surface, highlighting the physical structure, weave patterns and other characteristics of the fabric.

Before capturing each picture, the light conditions are optimized to guarantee that the brightness inside the hemispherical chamber is optimal, independently of the fabric's colour brightness. This process is possible because of the dome light system and grazing light system, which provide optimal illumination conditions inside the hemispherical diffused chamber light container. The brightness is controlled by the intensity of the light source in each of these bands (dome and grazing) and captured by the microscopic image device. The adaptation of the light source intensity is adjusted according to the fabric's colour general brightness. For instance, if a white coloured fabric is being analysed, the dome and grazing light source intensity should be lower than when analysing a dark coloured fabric. The hemispherical white and diffused light container causes an optimal and neutral diffusion distribution of light across the surface of the fabric. The light source intensity, targeting the optimal light conditions, is tunned using an algorithm that relies on the brightness level observed across the surface of the fabric or textile inside the hemispherical chamber.

After the light conditions are set, the picture is captured, and the analysis can be performed. Such analysis is made by a deep-learning architecture that receives both front and back images as inputs, and outputs the structure category.

The NIR spectrometer has a different mechanism as it is used for composition analysis. It is based in the absorption of light in the near infrared region of the electromagnetic spectrum (in this case, the NIR has a detecting region between 1550 and 1950 nm). The light is emitted towards the fabric, causing certain wavelengths to be absorbed more than others. The resulting absorption/reflection spectrums are differently shaped depending on the molecular components present in the fabric. Different compositions will result in a different absorption/reflection shape.

The overall system integrates both image and spectroscopic instruments and can be performed at the same time, as light emitted by the light source, and the NIR, do not affect each other measurements.

The manual process to estimate different parameters from the fabric is made in several ways. The process involves multiple steps that are time consuming and, in several cases, destructive. Regarding the fabric structure analysis, one of such inspections is made visually, by looking at the front and back of the fabric, the type of structure used can be estimated. This gave inspiration to study the usage of a vision-based solution, with a microscopic image device and a custom light system.

Regarding the composition analysis, the method is usually more time consuming and might involve tearing the fabric and burning segments of it. The yarns are weighed to obtain the percentage of incorporation of each one. A segment of mesh corresponding to 100 needles is then marked out and all the yarn removed. Each one is measured using a mesh meter. These measurements will give us the LFA of each yarn (yarn used in one round of the loom). Current state-of-the-art strategies used spectroscopic methods to estimate the composition of textile materials, especially in the near-infrared spectrum. Such study of the literature leads us to follow such approach. The advantages of using NIR regards the sensor availability, compact size, cost, easiness of integration into one system, and richness of the data collected.

The hardware is integrated into a single device that has a custom software and user interface. More details about the hardware used is presented in the technical report.

The software can be divided into two domains: (1) System control, interaction and processing, and (2) machine learning:

System Control, Interaction and Processing. The custom software was designed to control both camera + light system as well as the NIR spectrometer. Picture 3 shows the flow of system control during one acquisition step. Each mechanism can be developed separately (either structure or composition analysis), but the flow is the same. The steps are divided in A) System Configuration, B) Capture and C) Decision. During stage A, the models are loaded for future predictions and the elements are identified and turned on. The NIR system is calibrated with a white reference spectrum, and recording settings are configured.

After all these steps are made, the system can start a capture. For this, the user inputs a sample with the front face turned to the camera. From the camera side, the light system is adjusted to the colour brightness of the fabric and a capture is performed for both camera and NIR. After this, the user is required to flip the sample to face the back of the fabric. The same procedure is repeated (light adjustment and capture). In stage C), having the data captured and processed, the machine learning models are called for a prediction. The user is then asked to repeat a measurement or stop the analysis.

Machine Learning. The proposed invention is powered with artificial intelligence models. The software uses the pre-trained models and applies them to the collected data. Structure and composition use independent models.

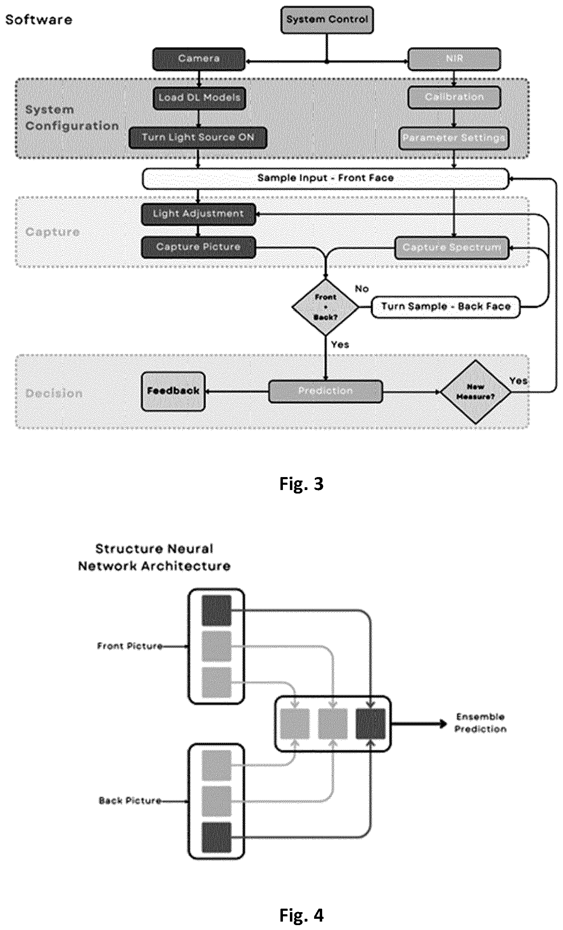

Regarding structure analysis, the deep learning architecture uses an innovative approach that follows a Siamese Convolutional Neural Network, combining the information of both front and back pictures, and an ensemble decision based on three models specifically trained with different training sets and different hyper-parameters. This process is depicted in **Figure 4****.**

Each model is Siamese, which means it has two main paths that independently accept a front and back picture of the fabric. In each path, the weights are combined to perform a common optimization. In addition, an ensemble of three models is used to make a prediction. This means that three different models (represented by a specific colour in **Figure 4**), are optimized independently, and their predictions are ensemble to give a more robust final prediction.

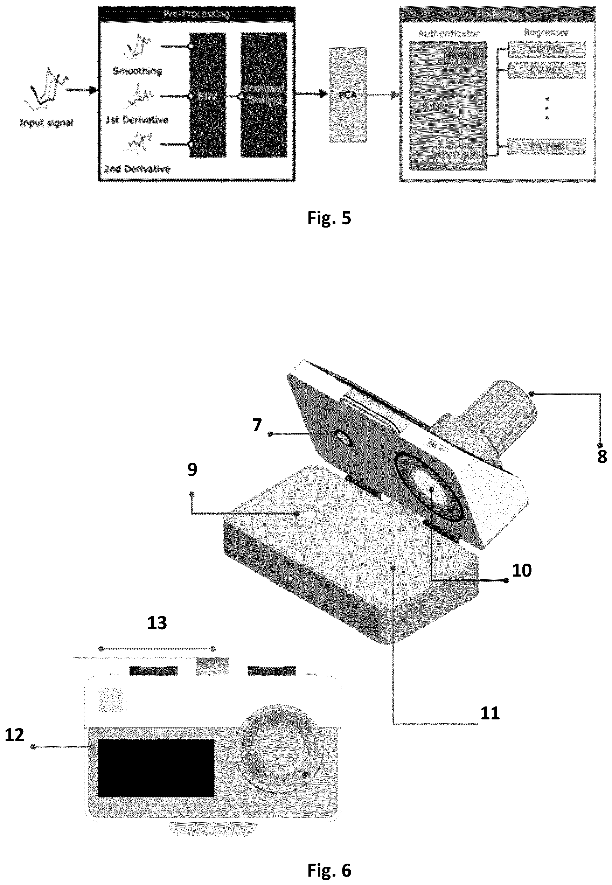

Regarding the composition estimation, an authenticator and specific regressor is applied to the incoming spectrum. First, it identifies the element(s) as a type of pure/mixture, and then, in case of being a mixture, performs a regression on this specific mixture type. As presented in **Figure 5****.**

In terms of structure analysis, the disclosure has utility in that:
- The current system is able to detect different types of structures, for example american fleece, jersey, 1x1 rib, 2x2 rib, interlock, interlock jaquard.
- The optimized lightning conditions can reduce the costs of the image capturing device, by using a standard and low-cost microscopic image device.

In terms of composition analysis, the disclosure has utility in that:
- The current system is able to distinguish different pure materials, for example CO, CV, PES, PA, CLY, WO, CL, PLA, EL, and other mixtures between these in specific percentage ranges, for example CO-EL; CO-PES ; CO-VISCOSE ; CO-PA ; CO-PLA ; CO-WO ; PES-WO ; PA-PES; PES-PLA; PES-EL ; VISCOSE-PA ; VISCOSE-PES ; VISCOSE-EL ; VISCOSE-WO ; ; PA-WO ; PA-EL.
- Front and back composition analysis are performed, giving more detailed information about the composition of the fabric

Additionally, the disclosure has utility in that:
- Both composition and structure information are provided in a standalone system;
- Possibility to select the type of analysis.

**Figure 6** shows a detailed technical drawing of a device for identifying structure and composition of a fabric or textile, where it can be seen **7** represents a white reference; **8** represents an adjustable camera; **9** represents a NIR spectrometer; **10** represents a microscope and light central system; **11** represents a data processor; **12** represents a touch screen display and **13** represents a tilt mechanism.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following claims further set out particular embodiments of the disclosure.

## Claims

1. Device for identifying structure and composition of a fabric or textile (5), comprising:
a chamber (6) for placing over the fabric or textile on a surface,
a diffused-light emitter (2) for illuminating the chamber with diffuse light;
a grazing-light emitter (3) for illuminating the fabric or textile with grazing light;
a camera (4) for acquiring images of the fabric or textile, mounted inside the chamber;
a near-infrared spectrometer for acquiring a near-infrared spectrum of the fabric or textile; and
a data processor;
wherein the device is configured for acquiring images of the fabric or textile;
wherein the data processor is arranged for carrying out the following steps:
activating the diffused-light emitter and the grazing-light emitter;
acquiring, from the camera, an image of the fabric or textile;
wherein the data processor is further arranged for carrying out the following steps:
acquiring, from the near-infrared spectrometer, a near-infrared spectrum of the fabric or textile;
using a pretrained classifier for classifying the structural identification and the composition of the textile or fabric, from the acquired image and the acquired spectrum.

2. Device according to the previous claim wherein the device is further configured for acquiring images of the fabric or textile at a plurality of rotation angles of the fabric or textile about an axis perpendicular to the surface.

3. Device according to the previous claim wherein the camera is rotatably mounted inside the chamber.

4. Device according to claim 2 wherein the chamber comprises a rotation union for rotating, relative to the fabric or textile, a part of the chamber where camera is mounted.

5. Device according to claim 3 or 4 wherein the camera or the part of the chamber is rotatable by a user of the device, in particular the chamber comprising a manual rotating mechanism for rotating the camera or the part of the chamber where camera is mounted, further in particular the chamber comprising a handle for rotating the camera or the part of the chamber where camera is mounted.

6. Device according to any of the claims 2-5, wherein the data processor is further arranged for detecting misalignment in the acquired image of the fabric or textile, between a structural direction of the fabric or textile and a reference direction, and if misalignment is detected, alerting a user of the device for re-acquiring, from the camera, an image of the fabric or textile rotated in respect of the previously acquired image about an axis perpendicular to the surface for aligning the structural direction of the fabric or textile with the reference direction.

7. Device according to claim 3 or 4, comprising a motor for rotating the camera or the part of the chamber where camera is mounted.

8. Device according to the previous claim, wherein the data processor is further arranged for:
detecting misalignment in the acquired image of the fabric or textile, between a structural direction of the fabric or textile and a reference direction;
if misalignment is detected, actuating the motor for rotating the camera or the part of the chamber where camera is mounted for aligning the structural direction of the fabric or textile with the reference direction; and
re-acquiring, from the camera, an image of the fabric or textile rotated in respect of the previously acquired image about an axis perpendicular to the surface.

9. Device according to claim 2 wherein the data processor is further arranged for:
detecting misalignment in the acquired image of the fabric or textile, between a structural direction of the fabric or textile and a reference direction;
if misalignment is detected, using digital image processing for rotating the acquired image for aligning the structural direction of the fabric or textile with the reference direction.

10. Device according to any of the previous claims wherein the chamber is a dome-shaped cover or a hemispherical-shaped cover.

11. Computer-based method for identifying structure and composition of a fabric or textile using a device according to any of the claims 1-10, the method comprising the following steps:
activating the diffused-light emitter and the grazing-light emitter;
acquiring, from the camera, an image of the fabric or textile;
wherein the method is further arranged for carrying out the following steps:
acquiring, from the near-infrared spectrometer, a near-infrared spectrum of the fabric or textile;
using a pretrained classifier for classifying the structural identification and the composition of the textile or fabric, from the acquired image and the acquired spectrum.

12. Method according to the previous claim wherein the device is further configured for acquiring images of the fabric or textile at a plurality of rotation angles of the fabric or textile about an axis perpendicular to the surface and said method is further arranged for detecting misalignment in the acquired image of the fabric or textile, between a structural direction of the fabric or textile and a reference direction, and if misalignment is detected, alerting a user of the device for re-acquiring, from the camera, an image of the fabric or textile rotated in respect of the previously acquired image about an axis perpendicular to the surface for aligning the structural direction of the fabric or textile with the reference direction.

13. Method according to claim 11 wherein said method is further arranged for:
detecting misalignment in the acquired image of the fabric or textile, between a structural direction of the fabric or textile and a reference direction;
if misalignment is detected, actuating a motor for rotating the camera or the part of the chamber where camera is mounted for aligning the structural direction of the fabric or textile with the reference direction; and re-acquiring, from the camera, an image of the fabric or textile rotated in respect of the previously acquired image about an axis perpendicular to the surface.

14. Method according to claim 11 wherein said method is further arranged for detecting misalignment in the acquired image of the fabric or textile, between a structural direction of the fabric or textile and a reference direction;
if misalignment is detected, using digital image processing for rotating the acquired image for aligning the structural direction of the fabric or textile with the reference direction.

15. Non-transitory storage media including program instructions for implementing a computer-based method for identifying structure and composition in a fabric or textile, the program instructions including instructions executable by a data processor to carry out the method of the claims 11-14.
